(19) Europäisches Patentamt
European Patent Office
Office européen des brevets

(11) **EP 4 065 186 B1**

(12) **EUROPEAN PATENT SPECIFICATION**

(45) Date of publication and mention
of the grant of the patent:
**05.07.2023 Bulletin 2023/27**

(21) Application number: **20811645.9**

(22) Date of filing: **27.11.2020**

(51) International Patent Classification (IPC):
*A61L 31/02* (2006.01)    *A61L 31/08* (2006.01)
*A61L 31/18* (2006.01)    *B32B 15/00* (2006.01)
*C23C 14/00* (2006.01)    *C23C 14/02* (2006.01)
*C23C 14/06* (2006.01)    *C23C 14/34* (2006.01)
*C23C 14/35* (2006.01)

(52) Cooperative Patent Classification (CPC):
**A61L 31/022; A61L 31/088; A61L 31/18;
C23C 14/0052; C23C 14/024; C23C 14/0641;
C23C 14/0676; C23C 14/3485; C23C 14/35;
A61L 2420/02; A61L 2420/06; A61L 2420/08**

(86) International application number:
**PCT/EP2020/083701**

(87) International publication number:
**WO 2021/105403 (03.06.2021 Gazette 2021/22)**

(54) **BIOCOMPATIBLE COATING FOR MEDICAL IMPLANT WITH TANTALUM RADIO-OPAQUE ADHESION LAYER**

BIOKOMPATIBLE BESCHICHTUNG FÜR MEDIZINISCHES IMPLANTAT MIT EINER RÖNTGENOPAKEN SCHICHT AUS TANTAL

REVÊTEMENT BIOCOMPATIBLE POUR IMPLANT MÉDICAL AVEC COUCHE D'ADHÉRENCE RADIO-OPAQUE AU TANTALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Validation States:
**MA**

(30) Priority: **29.11.2019 LU 101505**

(43) Date of publication of application:
**05.10.2022 Bulletin 2022/40**

(73) Proprietor: **IMC International Medical
Contrivances SA
1343 Luxembourg (LU)**

(72) Inventor: **MEKKI, Mustapha
1207 Genève (CH)**

(74) Representative: **Lecomte & Partners
76-78, rue de Merl
2146 Luxembourg (LU)**

(56) References cited:
**WO-A1-2011/104384    US-A1- 2005 165 472**

- **PARK CHEONIL ET AL: "Mechanically stable tantalum coating on a nano-roughened NiTi stent for enhanced radiopacity and biocompatibility", SURFACE AND COATINGS TECHNOLOGY, ELSEVIER BV, AMSTERDAM, NL, vol. 305, 6 August 2016 (2016-08-06), pages 139-145, XP029724264, ISSN: 0257-8972, DOI: 10.1016/J.SURFCOAT.2016.08.014**
- **YIFEI ZHANG ET AL: "Tantalum Nitride-Decorated Titanium with Enhanced Resistance to Microbiologically Induced Corrosion and Mechanical Property for Dental Application", PLOS ONE, vol. 10, no. 6, 24 June 2015 (2015-06-24), page e0130774, XP055720011, DOI: 10.1371/journal.pone.0130774**

- **FOX P ET AL: "Interface interactions between porous titanium/tantalum coatings, produced by Selective Laser Melting (SLM), on a cobalt-chromium alloy", SURFACE AND COATINGS TECHNOLOGY, ELSEVIER BV, AMSTERDAM, NL, vol. ti- and Ta- coatings on chromium based implants202, no. 20, 15 July 2008 (2008-07-15), pages 5001-5007, XP022777549, ISSN: 0257-8972, DOI: 10.1016/J.SURFCOAT.2008.05.003 [retrieved on 2008-05-13]**

## Description

## Technical field

The invention is directed to the field of coatings, more particularly biocompatible coatings for medical implants.

## Background art

Prior art patent document published WO 2011/104384 A1 discloses a surface coating for medical implants, and a method for applying such coating. The coating forms a single continuous layer along its thickness while comprises an inner portion metallic adhesion portion, an outer portion made of metallic oxynitrides and an intermediate portion made of metallic nitrides whose composition varies from the inner portion to the outer portion. This coating is interesting in that it provides a very good adhesion to the substrate even when the substrate, for instance a stent, is substantially deformed during use, for instance during implantation in the patient's body.

However, after implantation an implant needs to be checked by X-ray imaging. Also, it is now common to use biodegradable material, like PLLA (poly l-lactic acid) and magnesium, which are not visible with X-ray imaging, essentially due to their too low density.

For such radio invisible materials, it is known to attach small metallic chips or disks to end portions of a stent. This is however disadvantageous in that it increases the manufacturing costs, requires special care with cleaning the stent and chips prior to assembly, and also renders only the end portions of the stent visible with X-ray imaging.

## Summary of invention

### Technical Problem

The invention has for technical problem to overcome at least one drawback of the above cited prior art. More specifically, the invention has for technical problem to provide a biocompatible coating for medical implants that shows an increased adhesion. Even more specifically, the invention has for technical problem to provide biocompatible and radio-opaque medical implants that are also potentially biodegradable.

### Technical solution

The invention is directed to an implant comprising a substrate and a single layer coating on said substrate, said coating comprising: an innermost portion being a metallic ion-implanted adhesion portion; an outermost portion comprising metallic oxynitrides; and an intermediate portion between the innermost and outermost portions, said intermediate portion comprises metallic nitrides of different molar ratios; wherein the metal of the innermost portion is tantalum and said intermediate portion shows an average thickness of at least $1\,\mu$m, so as to be radio-opaque.

According to a preferred embodiment, the average thickness of the innermost portion of the single layer coating is not greater than $5\,\mu$m.

Advantageously, the average thickness of the innermost portion of the single layer coating is not greater than $3\,\mu$m.

According to a preferred embodiment, the metal of the metallic oxynitride of the outermost portion of the single layer coating, and/or of the metallic nitrides of the intermediate portion of the single layer coating is one or several solid transition metals of group IV B, V A, V B and VI B of the periodic table of the elements.

According to a preferred embodiment, the metal of the metallic oxynitride of the outermost portion of the single layer coating, and/or of the metallic nitrides of the intermediate portion of the single layer coating is titanium.

According to a preferred embodiment, the metal of the metallic oxynitride of the outermost portion of the single layer coating, and/or of the metallic nitrides of the intermediate portion of the single layer coating is tantalum.

According to a preferred embodiment, the substrate is made of a material selected from the following group: steel, preferably stainless steel and magnesium; nitinol; polymer.

According to a preferred embodiment, the metallic oxynitride of the outermost portion of the single layer coating has the general formula MNZOW, wherein Z+W=1 and Z is comprised between 0.2 to 0.9 with M being a solid transition metal, N being nitrogen and O being oxygen.

According to a preferred embodiment, the metallic nitrides of the intermediate portion of the single layer coating have the general formula MXNY with X+Y=1, X<1 and 0<Y<1, M being a solid transition metal and N being nitrogen.

The invention is also directed to a method for depositing a coating layer on a substrate, by Physical Vapour Deposition PVD where the substrate is enclosed in a chamber and subjected to bombardment by ions, comprising the following steps: bombarding metallic ions on the substrate while providing an inert gas in the chamber, so as to deposit an innermost portion of the coating layer, said portion being a metallic ion-implanted adhesion portion; bombarding metallic ions on the substrate while progressively providing gaseous nitrogen in the chamber, so as to deposit an intermediate portion of the coating layer, comprising metallic nitrides of different molar ratios; providing oxygen in the chamber while bombarding metallic ions on the substrate, so as to deposit an outermost portion of the coating layer, comprising metallic oxynitrides; wherein the metallic ions at step (a) are of tantalum, and the innermost portion of the coating layer shows an average thickness of at least $2\,\mu$m, so as to be radio-opaque.

According to a preferred embodiment, step (a) is achieved by High-power impulse magnetron sputtering HIPIMS.

According to a preferred embodiment, at step (c), oxygen is injected in the chamber so as to progressively replace the nitrogen provided at step (b).

According to a preferred embodiment, the metal of the metallic oxynitride of the outermost portion of the single layer coating, and/or of the metallic nitrides of the intermediate portion of the single layer coating is titanium, tantalum or a combination thereof.

The invention is also directed to an implant comprising a substrate and a single layer coating on said substrate, wherein said coating is obtainable by a method according to the invention.

Advantages of the invention

The invention is particularly interesting in that it provides an implant that is biocompatible and radio-opaque with a very good adhesion on many main materials of the implant, i.e. even if the main material of the implant is biodegradable and/or shows a low X-ray absorption coefficient, i.e. is radiolucent. The use of tantalum instead of titanium as metal in the innermost sub-layer or portion of the single coated layer provides non-only radio-opacity but also a better adhesion. The latter is also achieved thanks to the use of a particular PVD process, i.e. HIPIMS. The PVD process is particularly interesting in that it is carried out at low temperature, i.e. the substrate temperature does not exceed 100°C, meaning that the process is particularly adapted for non-metallic substrate like polymer (e.g. PLLA).

**Brief description of the drawings**

Figure 1 is a perspective view of a stent in a constrained configuration and in a deployed configuration.

Figure 2 is a schematic representation of a magnetron sputtering installation.

Figure 3 is a schematic cross-sectional view of the coating according to a first embodiment of the invention.

Figure 4 is a schematic cross-sectional view of the coating according to a second embodiment of the invention.

Figure 5 is a graphic showing the absorption coefficient of tantalum relative to the photon energy.

Figure 6 is picture of a portion of a stent coated with Titanium coated by Physical Vapour Deposition with direct current (DC).

Figure 7 is a magnification (about 5x) of the picture in figure 6.

Figure 8 is picture of a portion of a stent coated with Tantalum coated by High-Power Impulse Magnetron Sputtering (HiPIMS).

Figure 9 is a magnification (about 10x) of the picture in figure 8.

**Description of an embodiment**

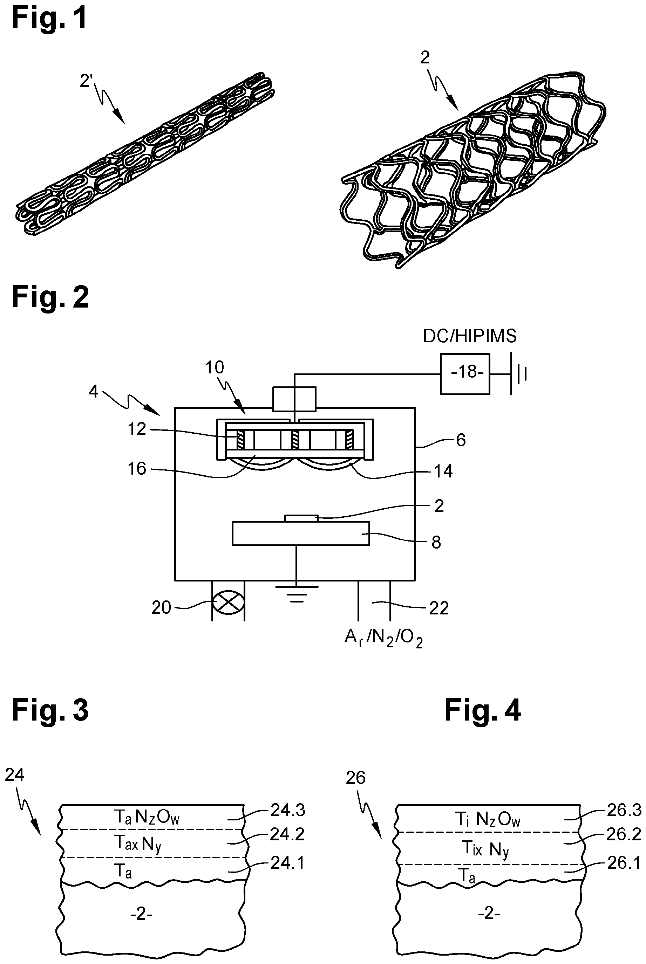

Figure 1 shows, at the right, a stent 2 in a deployed configuration and, at the left, the same stent 2' but in a constrained configuration. This illustrates the amplitude of elastic deformation that a stent can undergo during placement in a patient's body, e.g. in a coronary of a patient for coronary angioplasty.

The use of such stents is common since many years and has for major advantage that its placement into the patient's body is little invasive. The presence of a foreign element, often metallic, in the body can have undesirable side effects. It is however nowadays common to use biodegradable material, like poly-L-lactide (PLLA), for the stents in order to reduce these side-effects. Such material is not visible with X-ray imaging, essentially due to its too low density. Although biodegradable, such a material is not necessarily sufficiently biocompatible, i.e. shows a molecular outer surface that can have negative biological interactions with the host environment. It is therefore desired to provide a biocompatible coating on the main material of the stent. Accordingly, a biocompatible and radio-opaque coating is applied to the main material of the stent by a Physical Vapour Deposition (PVD) process.

Figure 2 is a schematic representation of a PVD installation, for instance a magnetron sputtering, or sputter deposition, installation 4. The installation comprises a chamber 6 that is hermetic to gas. An anode 8 is provided inside the chamber 6 and electrically connected to the ground. The anode 8 supports the substrate to be coated, for instance a stent 2 or any other implant or medical implant. A cathode 10 is also provided inside the chamber 6, in front of the surface of the anode 8 supporting the stent 2. The cathode 10 is for instance a magnetron one in that it comprises permanent magnets 12 that generate a permanent magnetic field 14. The cathode 10 carries a target 16 of the material to be coated on the stent 2. That material is tantalum. The cathode 10 is electrically connected to an electric generator 18, for instance of the DC (direct current) type or of the High-Power Impulse Magnetron Sputtering type (HIPIMS). HIPIMS utilises extremely high power densities in the order of $kW \cdot cm^{-2}$ in short pulses of tens of microsecond at low duty cycle (i.e. the on/on+off time ratio) of less than 10%.

The chamber 6 is connected to a vacuum pump 20 and comprises a gas feed port 22, for instance for Ar, $N_2$ and $O_2$.

The stent 2 is coated as follows. The chamber 6 is initially evacuated to high vacuum with the vacuum pump 18, to minimize the partial pressures of all background gases and potential contaminants. After base pressure has been reached, sputtering gas which comprises the plasma is flowed into the chamber and the total pressure is regulated - typically in the milliTorr range by using a pressure control system. A DC current of HIPIMS compliant current pulses are applied by the generator 18 to the cathode 10. Electrons which are present in the sputtering gas are accelerated away from the cathode causing collisions with nearby atoms of sputtering gas. These collisions cause an electrostatic repulsion which 'knock off' electrons from the sputtering gas atoms, causing ionization. The positive sputter gas atoms are now accelerated towards the negatively charged cathode, leading to high energy collisions with the surface of the target. Each of these collisions can cause atoms at the surface of the target to be ejected into the vacuum environment with enough kinetic energy to reach the surface of the substrate.

In a first phase, the chamber is filled with Argon (Ar) as sputtering gas in order to achieve a metallic ion-implanted adhesion innermost sub-layer, for instance a tantalum ion-implanted adhesion sub-layer. The pressure can be of about $10^{-3}$ hPa.

In a second phase, gaseous nitrogen ($N_2$) is injected into the chamber 6, thereby progressively replacing argon, by maintaining the total pressure at $10^{-3}$ hPa, so as to form a metallic nitride intermediate sub-layer where the metallic nitride has the general formula $M_X N_Y$ with X+Y=1, X<1 and 0<Y<1, M being a solid transition metal and N being nitrogen.

In a third phase, oxygen is injected into the chamber 6, thereby progressively replacing nitrogen by oxygen by maintaining the total pressure at $10^{-3}$ hPa, so as to form a metallic oxynitride outermost sub-layer, with the general formula $MN_Z O_W$, wherein Z+W=1 and Z is comprised between 0.2 to 0.9 with M being a solid transition metal, N being nitrogen and O being oxygen.

The resulting layer shows therefore three sub-layers or portions, i.e. an innermost portion, an intermediate portion and an outermost portion. The innermost portion is a tantalum ion-implanted adhesion portion, whereas the outermost portion is bioactive and biocompatible. The principle for achieving that resulting layer is as such known from WO 2011/104384 A1.

Figure 3 is schematic cross-section view of the single coated layer 24 according to a first embodiment, where the innermost portion 24.1 is made of tantalum, and the metal M of the nitride in the intermediate portion 24.2 and of the oxynitride in the outermost portion 24.3 is also tantalum.

Figure 4 is schematic cross-section view of the single coated layer 26 according to a second embodiment, where the innermost portion 26.1 is made of tantalum, and the metal M of the metallic nitride in the intermediate portion 26.2 and of the metallic oxynitride in the outermost portion 26.3 is titanium. This means that two cathodes with distinct target material, for instance tantalum and titanium are provided in the chamber of the installation.

In both embodiments, the innermost portion 24.1/26.1 of the coated single layer 24/26 shows two main advantageous properties, i.e. adhering and radio-opaque.

The thickness of the innermost portion 24.1/26.1 of the coated single layer 24/26 can be obtained as follows. In calculating a minimum thickness of the innermost portion of the coated layer for being radio-opaque, since X-rays shows wavelengths between 10nm and 0.01nm, we have to consider the whole range and therefore use these two limits. Considering the Planck-Einstein relation:

$$E = h \cdot \frac{c}{\lambda}$$

and that $\lambda_{min}$ = 0.01$nm$ and $\lambda_{max}$ = 10$nm$

we obtain that $E_{max}$ = 1.989 $\cdot$ 10$^{-14}J$ = 1.243 $\cdot$ 10$^{-1}eV$, and $E_{min}$ = 1.989 $\cdot$ 10$^{-17}J$ = 1.243 - 10$^{-4}eV$.

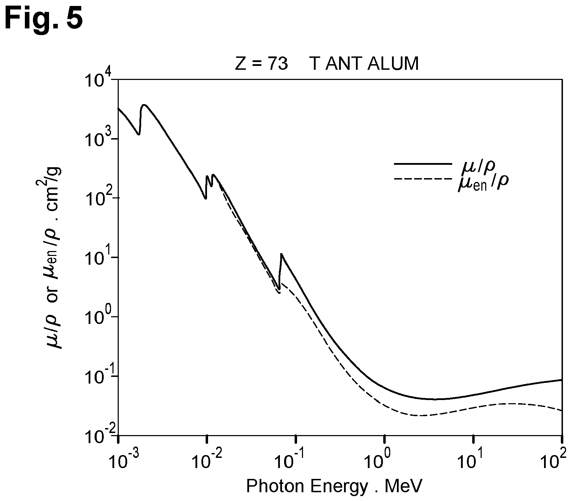

Using these values in the graphic in figure 5, we obtain the following mass absorption coefficients

$$\left(\frac{\mu}{\rho}\right)_{min} = 1.53 \ cm^2/g$$

$$\left(\frac{\mu}{\rho}\right)_{max} = 3.51 \cdot 10^3 \ cm^2/g$$

The mean free path is the average distance travelled by a moving particle, for instance a photon, between successive impacts or collisions which modify its direction or energy or other particle properties. The mean free path $l$ of a pencil beam of mono-energetic photons is the average distance a photon travels between collisions with atoms of the target material. It depends on the material and the energy of the photons and can be calculated as follows:

$$l = \frac{1}{\left(\frac{\mu}{\rho}\right) \cdot \rho} = \frac{1}{\mu}$$

It follows that $l_{average \ min}$ = 1.71$nm$ and $l_{average \ max}$ = 1.71$nm$.

Tests of radio-opacity on stents have been carried out, for instance by coating a tantalum layer of 300nm on a first stent and 2µm on a second stents. Each of these two stents has been inserted into a dead body, for instance a thoracic cage of a chicken. The chicken bodies have been X-ray imaged and the resulting images have been carefully analysed. The stent with the 300nm tantalum layer was not quite visible whereas the stent with the 2µm tantalum layer was well visible. We can then conclude that a thickness of at least 1 µm, preferably at least 2µm, is satisfying for achieving radio-opacity.

Adhesion tests have been carried out by closely observing the surface roughness of a first stent coated according to the prior art WO 2011/104384 A1, i.e. with a titanium innermost portion of the single layer coating, and a second sent coated according the invention. The first stent is made of stainless steel 316L and is coated by PVD DC with an innermost portion of the single layer made of titanium with a thickness of 50nm. The second stent is identical to the first one and is coated by HIPIMS with an innermost portion of the single layer made of tantalum with a thickness of 50nm.

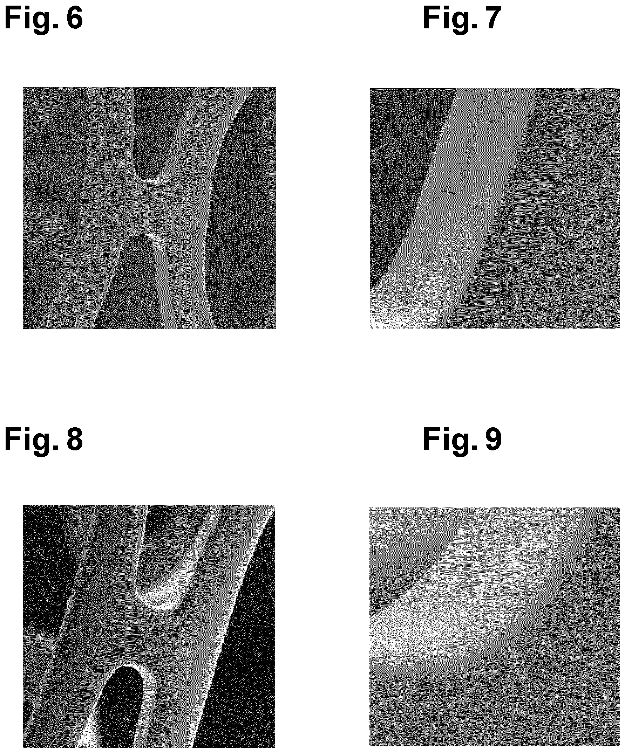

Figures 6 and 7 show the first stent, i.e. coated according the prior art. In figure 7 which is a magnified view (at a magnification level of x1000) of a portion of figure 6, we can see slight cracks but no chipping of the coating, meaning that the adhesion is readily satisfying.

Figures 8 and 9 show the second sent, i.e. coated according to the invention. In figure 9 which is a magnified view (at a magnification level of x1000) of a portion of figure 8, we can see a smooth surface with no cracks, meaning that the result is substantially better as in the prior art.

Further adhesion tests have been carried out on stents made of stainless steel 316L, Cobalt alloy L605 and Magnesium alloy. Each of them has been coated according to the invention, for instance with Ta/TiNO, in a compressed or unexpanded state. The stents are then expanded by means of a balloon catheter and an inflator fluidly connected thereto. For instance, an inflator of the company Boston Scientific, model Encore® 26, has been used. The pressures applied are according to the nominal pressures provided in a table relating with the balloon catheter. With reference to figures 1 and 2, each of the test stents has been brought from the compressed state as illustrated in figure 1, to the deployed or expanded state as illustrated in figure 2. After complete deployment, each stent has been carefully examined with an Electronic Scanning Microscope, i.e. with magnification levels of x250, x1000, x3000 and x5000. On certain stents, slight crack initiations were visible at the highest magnification level, i.e. x5000, but such defaults are considered acceptable in that they do not lead to chipping of the coating.

## Claims

1. Implant (2) comprising a substrate and a single layer coating (24; 26) on said substrate, said coating comprising:

   - an innermost portion (24.1; 26.1) being a metallic ion-implanted adhesion portion;
   - an outermost portion (24.3; 26.3) comprising metallic oxynitrides; and
   - an intermediate portion (24.2; 26.2) between the innermost and outermost portions (24.1, 24.3; 26.1, 26.3), said intermediate portion comprises metallic nitrides of different molar ratios;

   **characterized in that**
   the metal of the innermost portion (24.1; 26.1) is tantalum and said portion shows an average thickness of at least 1µm, so as to be radio-opaque.

2. Implant (2) according to claim 1, wherein the average thickness of the innermost portion (24.1; 26.1) of the single layer coating (24; 26) is not greater than 5µm.

3. Implant (2) according to one of claims 1 and 2, wherein the metal of the metallic oxynitride of the outermost portion (24.3; 26.3) of the single layer coating (24; 26), and/or of the metallic nitrides of the intermediate portion (24.2; 26.2) of the single layer coating (24; 26) is one or several solid transition metals of group IV B, V A, V B and VI B of the periodic table of the elements.

4. Implant (2) according to one of claims 1 and 2, wherein the metal of the metallic oxynitride of the outermost portion (26.3) of the single layer coating (26), and/or of the metallic nitrides of the intermediate portion (26.2) of the single layer coating (26) is titanium.

5. Implant (2) according to one of claims 1 and 2, wherein the metal of the metallic oxynitride of the outermost portion (24.3) of the single layer coating (24), and/or of the metallic nitrides of the intermediate portion (24.2) of the single layer coating (24) is tantalum.

6. Implant (2) according to any one of claims 1 to 5, wherein the substrate is made of a material selected from the following group: steel, preferably stainless steel and magnesium; nitinol; polymer.

7. Implant (2) according to any one of claims 1 to 6, wherein the metallic oxynitride of the outermost portion (24.3; 26.3) of the single layer coating (24; 26) has the general formula $MN_ZO_W$, wherein Z+W=1 and Z is comprised between 0.2 to 0.9 with M being a solid transition metal, N being nitrogen and O being oxygen.

8. Implant (2) according to any one of claims 1 to 7, wherein the metallic nitrides of the intermediate portion (24.2; 26.2) of the single layer coating (24; 26) have the general formula $M_X N_Y$ with X+Y=1, X<1 and 0<Y<1, M being a solid transition metal and N being nitrogen.

9. Method for depositing a coating a single layer (24; 26) on a substrate, by Physical Vapour Deposition PVD where the substrate is enclosed in a chamber (6) and subjected to bombardment by ions, comprising the following steps:

(a) bombarding metallic ions on the substrate while providing an inert gas in the chamber (6), so as to deposit an innermost portion (24.1; 26.1) of the coating layer, said portion being a metallic ion-implanted adhesion portion;
(b) bombarding metallic ions on the substrate while progressively providing gaseous nitrogen in the chamber (6), so as to deposit an intermediate portion (24.2; 26.2) of the coating layer (24; 26), comprising metallic nitrides of different molar ratios;
(c) providing oxygen in the chamber (6) while bombarding metallic ions on the substrate, so as to deposit an outermost portion (24.3; 26.3) of the coating layer (24; 26), comprising metallic oxynitrides;

**characterized in that**
the metallic ions at step (a) are of tantalum, and the innermost portion (24.1; 26.1) of the coating layer shows an average thickness of at least 2µm, so as to be radio-opaque.

10. Method according to claim 9, wherein step (a) is achieved by High-power impulse magnetron sputtering HIPIMS.

11. Method according to one of claims 9 and 10, wherein at step (c), oxygen is injected in the chamber so as to progressively replace the nitrogen provided at step (b).

12. Method according to one of claims 9 to 11, wherein the metal of the metallic oxynitride of the outermost portion (24.3; 26.3) of the single layer coating (24; 26), and/or of the metallic nitrides of the intermediate portion (24.2; 26.2) of the single layer coating (24; 26) is titanium, tantalum or a combination thereof.

13. Implant (2) comprising a substrate and a single layer coating (24; 26) on said substrate, **characterized in that** said single layer coating (24; 26) is obtainable by a method according to any one of claims 9-12.

**Patentansprüche**

1. Implantat (2), umfassend ein Substrat und eine einschichtige Beschichtung (24; 26) auf dem Substrat, wobei die Beschichtung umfasst:

- ein innerster Abschnitt (24.1; 26.1), der ein metallischer, ionenimplantierter Adhäsionsabschnitt ist;
- einen äußersten Abschnitt (24.3; 26.3), der metallische Oxynitride umfasst; und
- einen Zwischenabschnitt (24.2; 26.2) zwischen dem innersten und dem äußersten Abschnitt (24.1, 24.3; 26.1, 26.3), wobei der Zwischenabschnitt Metallnitride unterschiedlicher Molverhältnisse umfasst;

**dadurch gekennzeichnet**
das Metall des innersten Abschnitts (24.1; 26.1) Tantal ist und besagter Abschnitt eine durchschnittliche Dicke von mindestens 1 µm aufweist, so dass er strahlenundurchlässig ist.

2. Implantat (2) nach Anspruch 1, wobei die durchschnittliche Dicke des innersten Abschnitts (24.1; 26.1) der Einzelschichtbeschichtung (24; 26) nicht größer als 5 µm ist.

3. Implantat (2) nach einem der Ansprüche 1 und 2, wobei das Metall des metallischen Oxynitrids des äüersten Abschnitts (24.3; 26.3) der einschichtigen Beschichtung (24; 26) und/oder der metallischen Nitride von der Zwischenabschnitt (24.2; 26.2) der einschichtigen Beschichtung (24; 26) ein oder mehrere feste Übergangsmetalle der Gruppe IV B, V A, V B und VI B des Periodensystems der Elemente ist.

4. Implantat (2) nach einem der Ansprüche 1 und 2, wobei das Metall des Metalloxynitrids des äußersten Abschnitts (26.3) der einschichtigen Beschichtung (26) und/oder der Metallnitride des Zwischenabschnitts ( 26.2) der einschichtigen Beschichtung (26) Titan ist.

5. Implantat (2) nach einem der Ansprüche 1 und 2, wobei das Metall des metallischen Oxynitrids des äüersten Abschnitts (24.3) der einschichtigen Beschichtung (24) und/oder der metallischen Nitride des Zwischenabschnitts (24.2) der einschichtigen Beschichtung (24) Tantal ist.

6. Implantat (2) nach einem der Ansprüche 1 bis 5, wobei das Substrat aus einem Material ausgewählt aus der folgenden Gruppe besteht: Stahl, vorzugsweise Edelstahl und Magnesium; Nitinol; Polymer.

7. Implantat (2) nach einem der Ansprüche 1 bis 6, wobei das metallische Oxynitrid des äußersten Abschnitts (24.3; 26.3) der einschichtigen Beschichtung (24; 26) die allgemeine Formel MNzOw aufweist, wobei Z+W= 1 und Z liegt zwischen 0,2 und 0,9, wobei M ein festes Übergangsmetall ist, N Stickstoff und O Sauerstoff ist.

8. Implantat (2) nach einem der Ansprüche 1 bis 7, wobei die metallischen Nitride des Zwischenabschnitts (24.2; 26.2) der einschichtigen Beschichtung (24; 26) die allgemeine Formel $M_XN_Y$ mit X+Y=1 aufweisen, X<1 und 0<Y<1, wobei M ein festes Übergangsmetall und N Stickstoff ist.

9. Verfahren zum Aufbringen einer einschichtigen Beschichtung (24; 26) auf einem Substrat durch physikalische Gasphasenabscheidung (PVD), wobei das Substrat in einer Kammer (6) eingeschlossen und einem Ionenbeschuss ausgesetzt wird, umfassend die folgenden Schritte:

(a) Beschießen des Substrats mit metallischen Ionen unter Bereitstellung eines Inertgases in der Kammer (6), um einen innersten Abschnitt (24.1; 26.1) der Beschichtungsschicht abzuscheiden, wobei dieser Abschnitt ein mit metallischen Ionen implantierter Adhäsionsabschnitt ist;
(b) Beschießen des Substrats mit Metallionen unter fortschreitender Bereitstellung von gasförmigem Stickstoff in der Kammer (6), um so einen Zwischenabschnitt (24.2; 26.2) der Beschichtungsschicht (24; 26) abzuscheiden, der Metallnitride unterschiedlicher Molverhältnisse umfasst;
(c) Bereitstellen von Sauerstoff in der Kammer (6), während Metallionen auf das Substrat bombardiert werden, um so einen äußersten Teil (24.3; 26.3) der Beschichtungsschicht (24; 26) abzuscheiden, die metallische Oxynitride umfasst;

**dadurch gekennzeichnet**
die Metallionen in Schritt (a) aus Tantal bestehen und der innerste Teil (24.1; 26.1) der Beschichtungsschicht eine durchschnittliche Dicke von mindestens 2 $\mu$m aufweist, um strahlenundurchlässig zu sein.

10. Verfahren nach Anspruch 9, wobei Schritt (a) durch Hochleistungs-Impulsmagnetronsputtern von HIPIMS erreicht wird.

11. Verfahren nach einem der Ansprüche 9 und 10, wobei in Schritt (c) Sauerstoff in die Kammer injiziert wird, um den in Schritt (b) bereitgestellten Stickstoff schrittweise zu ersetzen.

12. Verfahren nach einem der Ansprüche 9 bis 11, wobei das Metall des metallischen Oxynitrids des äußersten Abschnitts (24.3; 26.3) der einschichtigen Beschichtung (24; 26) und/oder des metallischen Nitrids des Zwischenabschnitts ist (24.2; 26.2) der Einzelschichtbeschichtung (24; 26) Titan, Tantal oder eine Kombination davon ist.

13. Implantat (2), umfassend ein Substrat und eine einschichtige Beschichtung (24; 26) auf dem Substrat, **dadurch gekennzeichnet, dass** die einschichtige Beschichtung (24; 26) durch ein Verfahren nach einem der Ansprüche 9-12 erhältlich ist.

**Revendications**

1. Implant (2) comprenant un substrat et un revêtement monocouche (24 ; 26) sur ledit substrat, ledit revêtement comprenant :

- une partie la plus interne (24.1; 26.1) étant une partie d'adhésion à ions métalliques implantés ;
- une partie la plus externe (24.3; 26.3) comprenant des oxynitrures métalliques ; et
- une partie intermédiaire (24.2; 26.2) entre les parties la plus interne et la plus externe (24.1, 24.3 ; 26.1, 26.3), ladite partie intermédiaire comprenant des nitrures métalliques de rapports molaires différents ;

**caractérisé en ce que**
le métal de la partie la plus interne (24.1; 26.1) est du tantale et ladite partie présente une épaisseur moyenne d'au moins 1 $\mu$m, de manière à être radioopaque.

2. Implant (2) selon la revendication 1, dans lequel l'épaisseur moyenne de la partie la plus interne (24.1; 26.1) du revêtement monocouche (24; 26) n'est pas supérieure à 5 $\mu$m.

3. Implant (2) selon l'une des revendications 1 et 2, dans lequel le métal de l'oxynitrure métallique de la partie la plus externe (24.3; 26.3) du revêtement monocouche (24; 26), et/ou des nitrures métalliques de la partie intermédiaire (24.2 ; 26.2) du revêtement monocouche (24; 26) est un ou plusieurs métaux de transition solides des groupes IV B, V A, V B et VI B du tableau périodique des éléments.

4. Implant (2) selon l'une des revendications 1 et 2, dans lequel le métal de l'oxynitrure métallique de la partie la plus externe (26.3) du revêtement monocouche (26), et/ou des nitrures métalliques de la partie intermédiaire (26.2) du revêtement monocouche (26) est du titane.

5. Implant (2) selon l'une des revendications 1 et 2, dans lequel le métal de l'oxynitrure métallique de la partie la plus externe (24.3) du revêtement monocouche (24), et/ou des nitrures métalliques de la partie intermédiaire (24.2) du revêtement monocouche (24) est en tantale.

6. Implant (2) selon l'une quelconque des revendications 1 à 5, dans lequel le substrat est réalisé en un matériau choisi dans le groupe suivant : acier, de préférence acier inoxydable et magnésium; nitinol; polymère.

7. Implant (2) selon l'une quelconque des revendications 1 à 6, dans lequel l'oxynitrure métallique de la partie la plus externe (24.3 ; 26.3) du revêtement monocouche (24 ; 26) a la formule générale MNzOw, dans laquelle Z+W= 1 et Z est compris entre 0,2 et 0,9, M étant un métal de transition solide, N étant l'azote et O étant l'oxygène.

8. Implant (2) selon l'une quelconque des revendications 1 à 7, dans lequel les nitrures métalliques de la partie intermédiaire (24.2 ; 26.2) du revêtement monocouche (24 ; 26) ont pour formule générale $M_X$-$N_Y$ avec X+Y=1, X<1 et 0<Y<1, M étant un métal de transition solide et N étant l'azote.

9. Procédé de dépôt d'un revêtement monocouche (24 ; 26) sur un substrat, par dépôt physique en phase vapeur PVD où le substrat est enfermé dans une enceinte (6) et soumis à un bombardement par des ions, comprenant les étapes suivantes :

   (a) bombarder des ions métalliques sur le substrat tout en fournissant un gaz inerte dans la chambre (6), de manière à déposer une partie la plus interne (24.1 ; 26.1) de la couche de revêtement, ladite partie étant une partie d'adhérence implantée d'ions métalliques ;
   (b) bombarder des ions métalliques sur le substrat tout en fournissant progressivement de l'azote gazeux dans la chambre (6), de manière à déposer une partie intermédiaire (24.2 ; 26.2) de la couche de revêtement (24 ; 26), comprenant des nitrures métalliques de différents rapports molaires ;
   (c) fournir de l'oxygène dans la chambre (6) tout en bombardant des ions métalliques sur le substrat, de manière à déposer une partie la plus externe (24.3 ; 26.3) de la couche de revêtement (24 ; 26), comprenant des oxynitrures métalliques ;

   **caractérisé en ce que**
   les ions métalliques à l'étape (a) sont en tantale, et la partie la plus interne (24.1 ; 26.1) de la couche de revêtement présente une épaisseur moyenne d'au moins 2 $\mu$m, de manière à être radio-opaque.

10. Procédé selon la revendication 9, dans lequel l'étape (a) est réalisée par pulvérisation magnétron impulsionnelle de forte puissance HIPIMS.

11. Procédé selon l'une des revendications 9 et 10, dans lequel à l'étape (c), de l'oxygène est injecté dans l'enceinte de manière à remplacer progressivement l'azote apporté à l'étape (b).

12. Procédé selon l'une des revendications 9 à 11, dans lequel le métal de l'oxynitrure métallique de la partie la plus externe (24.3 ; 26.3) du revêtement monocouche (24 ; 26), et/ou des nitrures métalliques de la partie intermédiaire (24.2 ; 26.2) du revêtement monocouche (24 ; 26) est du titane, du tantale ou une combinaison de ceux-ci.

13. Implant (2) comprenant un substrat et un revêtement monocouche (24 ; 26) sur ledit substrat, **caractérisé en ce que** ledit revêtement monocouche (24 ; 26) peut être obtenu par un procédé selon l'une quelconque des revendications 9-12.

# Fig. 1

# Fig. 2

# Fig. 3

# Fig. 4

# Fig. 5

Z = 73    T ANT ALUM

Fig. 6

Fig. 7

Fig. 8

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011104384 A1